# EUROPEAN PATENT APPLICATION

(11) **EP 4 129 971 A1**
(43) Date of publication of application: **08.02.2023**
(21) Application number: 21774539.7
(22) Date of filing: 23.03.2021
(51) Int. Cl.: C07C 59/66, C07C 309/11, C07C 323/66, C07C 49/755, H01M 8/02, H01M 8/18

(54) **HETEROCYCLIC COMPOUND OR SALT THEREOF, ACTIVE MATERIAL, ELECTROLYTE AND REDOX FLOW BATTERY**

(30) Priority: 24.03.2020 JP 2020052173; 07.10.2020 JP 2020169567; 18.01.2021 JP 2021005637; 18.01.2021 JP 2021005638
(71) Applicant: Nippon Kayaku Kabushiki Kaisha, Tokyo 100-0005 (JP)
(72) Inventor: KAIHO Atsushi, Tokyo 115-8588 (JP); NAGATSUKA Shinya, Tokyo 115-8588 (JP); NAKAJIMA Tomoya, Tokyo 115-8588 (JP)
(74) Representative: Hentrich Patent- & Rechtsanwälte PartG mbB
(86) International application number: PCT/JP2021/011880
(87) International publication number: WO 2021/193594

(57) **Abstract**

The present invention relates to a heterocyclic compound represented by formula (1), (2) or (3), or a salt thereof. The present invention also relates to an active material containing at least one heterocyclic compound or a salt thereof described above, an electrolytic solution containing the active material, and a redox flow battery including the electrolytic solution.

## Description

### Technical Field

The present invention relates to a heterocyclic compound and a salt thereof. The present invention also relates to an active material containing the heterocyclic compound or a salt thereof, an electrolytic solution containing the active material, and a redox flow battery including the electrolytic solution.

### Background Art

Along with the increase of capacity of facilities using renewable energy, the introduction of large storage batteries is progressing in order to stabilize the system power. Electrolytic solutions of redox flow batteries which are expected to serve as large storage batteries include aqueous ones and nonaqueous ones, but aqueous electrolytic solutions are superior in the safety and the cost. Hence, active materials are required to have a high solubility to water and in order to attain a high energy density, are desired to have a suitable oxidation reduction potential.

For currently leading redox flow batteries, vanadium is used as an active material. Use of vanadium, however, has resource restrictions and the problem of fluctuation of the price (Non Patent Literatures 1, 2). Hence, for active materials, use of materials abundant as resources is desirable.

### Document List

### Non Patent Literatures

Non Patent Literature 1: Jan Winsberg et al., Angew. Chem. Int. Ed. 2017, 56, 686-711
Non Patent Literature 2: P. Leung et al., Journal of Power Sources 360 (2017) 243-283

### Summary of Invention

### Technical Problem

The present invention has an object to provide a novel compound, an active material, an electrolytic solution and a redox flow battery which enable the improvement of the energy density and the cycle characteristic of the redox flow battery using an aqueous electrolyte solution.

### Solution to Problem

A heterocyclic compound or a salt thereof according to an aspect of the present invention is represented by the following formula (1), (2) or (3): 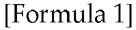 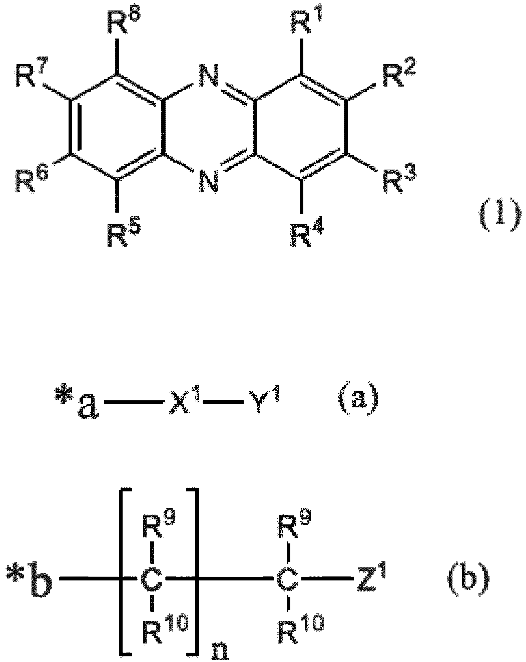 wherein:
R¹ to R⁸ are each independently a hydrogen atom, an acidic group, an alkoxy group, an alkyl group, an amino group, an amide group or a group represented by formula (a), and at least one of R¹ to R⁸ is a group represented by formula (a);
X¹ is an oxygen atom, a sulfur atom or NY²;
Y¹ is a group represented by formula (b);
Y² is a hydrogen atom, an alkyl group, a carbonyl group, a sulfonyl group or a group represented by formula (b);
R⁹ and R¹⁰ each independently represent a hydrogen atom or a substituent;
Z¹ is an acidic group;
n represents an integer of 1 to 7;
*a represents a bonding site to formula (1); and
*b represents a bonding site to X¹ of formula (a), 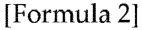 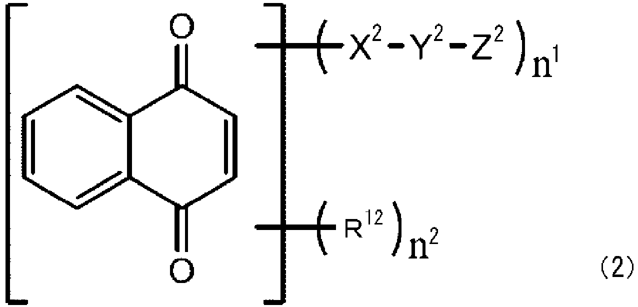 wherein:
   X² is an oxygen atom, a sulfur atom or NR¹¹;
   Y² is a linker;
   Z² is an acidic group;
   n¹ is an integer of 1 to 6;
   R¹¹ is a hydrogen atom or an alkyl group;
   each R¹² is independently an alkyl group or an acidic group; and
   n² is an integer of 0 to 5, and 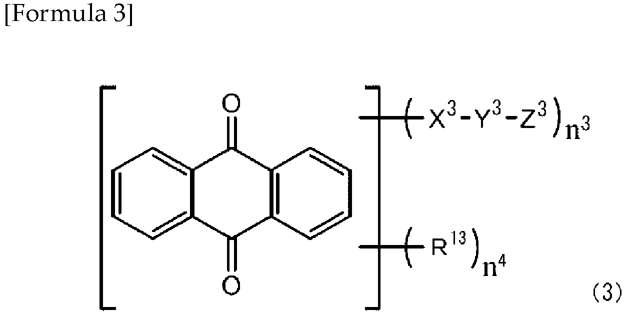 wherein:
      X³ is an oxygen atom or a sulfur atom;
      Y³ is a linker;
      Z³ is an acidic group;
      n³ is an integer of 3 to 8;
      each R¹³ is independently an alkyl group or an acidic group; and n⁴ is an integer of 0 to 5.

In one embodiment of the present invention, Z¹ in the above formula (b) is a sulfo group.

In one embodiment of the present invention, at least two of R¹ to R⁸ in the above formula (1) are each a group represented by the above formula (a).

In one embodiment of the present invention, R² and R³ in the above formula (1) are each a group represented by the above formula (a).

An active material according to an aspect of the present invention contains at least one heterocyclic compound or a salt thereof described above.

An electrolytic solution according to an aspect of the present invention contains the above active material.

In one embodiment of the present invention, the electrolytic solution is an electrolytic solution for a redox flow battery.

In one embodiment of the present invention, the content of water contained in the electrolytic solution is 1% by mass or higher and 99.99% by mass or lower.

In one embodiment of the present invention, the content of water contained in the electrolytic solution is 10% by mass or higher and 99% by mass or lower.

A redox flow battery according to an aspect of the present invention includes the above electrolytic solution.

In one embodiment of the present invention, the redox flow battery further includes an electrode and a membrane.

### Effects of Invention

According to the present invention, there can be realized a novel compound, an active material, an electrolytic solution and a redox flow battery which enable the improvement of the energy density and the cycle characteristic of the redox flow battery using an aqueous electrolytic solution.

### Brief Description of Drawings

[Fig. 1] A charge/discharge curve diagram exhibited by a redox flow battery 1 fabricated in Example 4.
[Fig. 2] A charge/discharge curve diagram exhibited by a redox flow battery 2 fabricated in Example 5.
[Fig. 3] A charge/discharge curve diagram exhibited by a redox flow battery 3 fabricated in Example 6.
[Fig. 4] A charge/discharge curve diagram exhibited by a redox flow battery 4 fabricated in Example 9.
[Fig. 5] A charge/discharge curve diagram exhibited by a redox flow battery 5 fabricated in Example 12.
[Fig. 6] A charge/discharge curve diagram exhibited by a redox flow battery 6 fabricated in Example 13.
[Fig. 7] A charge/discharge curve diagram exhibited by a redox flow battery 7 fabricated in Example 15.
[Fig. 8] A charge/discharge curve diagram exhibited by a redox flow battery 8 fabricated in Example 22.
[Fig. 9] A charge/discharge curve diagram exhibited by a redox flow battery 9 fabricated in Example 23.
[Fig. 10] A charge/discharge curve diagram exhibited by a redox flow battery 10 fabricated in Example 24.
[Fig. 11] A charge/discharge curve diagram exhibited by a redox flow battery 11 fabricated in Example 25.
[Fig. 12] A charge/discharge curve diagram exhibited by a redox flow battery 12 fabricated in Example 26.
[Fig. 13] A charge/discharge curve diagram exhibited by a redox flow battery 13 fabricated in Example 27.
[Fig. 14] A charge/discharge curve diagram exhibited by a redox flow battery 14 fabricated in Comparative Example 1.
[Fig. 15] A charge/discharge curve diagram exhibited by a redox flow battery 15 fabricated in Comparative Example 2.
[Fig. 16] A charge/discharge curve diagram exhibited by a redox flow battery 16 fabricated in Comparative Example 3.

### Description of Embodiments

Hereinafter, the present invention will be described in detail. In the present description, including Examples and the like, unless otherwise specified, "parts" and "%" are both in terms of mass. Further, in order to avoid complexity, the description of a "compound or a salt thereof" is, for convenience, simply described to be a "compound" in some cases.

### <Heterocyclic compound>

A heterocyclic compound according to the present embodiments is represented by the following formula (1), (2) or (3). That is, the heterocyclic compound is selected from the group consisting of phenazine-based compounds represented by the following formula (1), naphthoquinone-based compounds represented by the following formula (2) and anthraquinone-based compounds represented by the following formula (3). By an aqueous electrolytic solution for a redox flow battery containing such a heterocyclic compound as an active material, the energy density and the cycle characteristic of the redox flow battery can be improved. In the case where these compounds form a salt, the salt may be, for example, an alkaline metal salt such as a lithium salt, a sodium salt or a potassium salt, an alkaline earth metal salt such as a calcium salt or an ammonium salt such as an ammonium salt and a tetramethylammonium salt. Then in the case where a compound represented by the formula (1), formula (2) or formula (3) has a plurality of acidic groups, these groups may be all free acids, all salts, or partially free acids (partially salts). In the case where in these compounds, a plurality of salts are present, these salts may be ones of the same kind, or may be ones of different kinds.

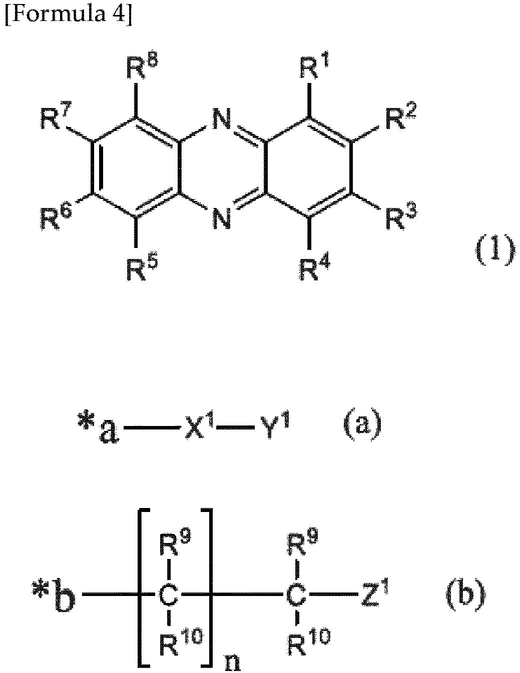

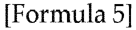

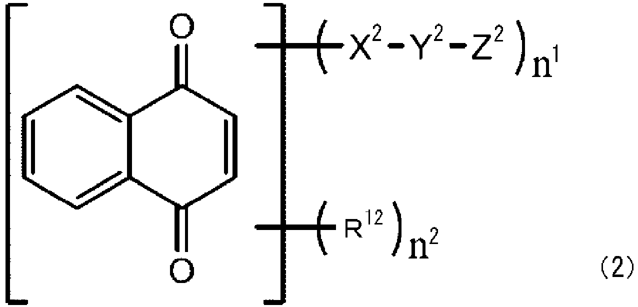

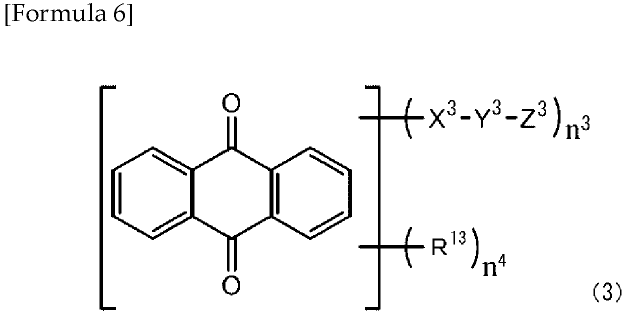

### [Phenazine-based compounds represented by the formula (1)]

In the formula (1), R¹ to R⁸ are each independently a hydrogen atom, an acidic group, an alkoxy group, an alkyl group, an amino group, an amide group or a group represented by the formula (a), and at least one of R¹ to R⁸ is a group represented by the formula (a); X¹ in the formula (a) is an oxygen atom, a sulfur atom or NY²; Y¹ is a group represented by the formula (b); Y² is a hydrogen atom, an alkyl group, a carbonyl group, a sulfonyl group or a group represented by the formula (b); R⁹ and R¹⁰ in the formula (b) each independently represent a hydrogen atom or a substituent; Z¹ is an acidic group; n is an integer of 1 to 7; *a in the formula (a) represents a bonding site to the formula (1); and *b in the formula (b) represents a bonding site to X¹ of the formula (a).

Examples of the acidic group include a sulfo group, a carboxy group, a phosphoric acid group and a hydroxy group, and a sulfo group is preferable. These acidic groups may be free acids or may form salts.

Examples of the alkoxy group include a methoxy group, an ethoxy group, a n-propoxy group, an isopropoxy group, a n-butoxy group and a t-butoxy group.

Examples of the amino group include an amino group (-NH₂), a methylamino group, a dimethylamino group, an ethylamino group and a diethylamino group, and an amino group and the like are preferable.

Examples of the amide group include a formamide group, an acetoamide group, a benzamide group and a pivalamide group, and an acetoamide group and the like are preferable.

Examples of the alkyl group include a methyl group, an ethyl group, a n-propyl group, an isopropyl group, a n-butyl group and a t-butyl group.

Examples of the carbonyl group include an acetyl group, a pivaloyl group and a benzoyl group, and an acetyl group and the like are preferable.

Examples of the sulfonyl group include a methanesulfonyl group, a p-toluenesulfonyl group, an o-nitrobenzenesulfonyl group and a trifluoromethanesulfonyl group.

Examples of the substituent include an alkyl group.

It is preferable that n is 1 to 6; being 1 to 3 is more preferable; being 1 or 2 is still more preferable; and being 2 is especially preferable.

It is preferable that at least two of R¹ to R⁸ in the formula (1) are each a group represented by the above formula (a); and it is more preferable that R² and R³ are each a group represented by the above formula (a). It is preferable that Z¹ in the formula (b) is a sulfo group.

As a specific form of the phenazine-based compound represented by the formula (1), it is preferable that R² is a group represented by the above formula (a); R³ is a group represented by the formula (a) or an acidic group; R⁶ is a hydrogen atom, an amide group, an amino group or an acidic group; R¹, R⁴, R⁵, R⁷ and R⁸ are each a hydrogen atom; X¹ in the formula (a) is an oxygen atom; R⁹ and R¹⁰ in the formula (b) are each a hydrogen atom; and n is 2.

The phenazine-based compound represented by the formula (1) may be used singly in one kind, or may be used in a combination of two or more kinds. In the case of using by combining two or more kinds, the two or more kinds can be used concurrently in any proportion.

### [Naphthoquinone-based compound represented by the formula (2)]

In the formula (2), X² is an oxygen atom, a sulfur atom or NR¹¹; Y² is a linker; Z² is an acidic group; n¹ is an integer of 1 to 6; R¹¹ is a hydrogen atom or an alkyl group; each R¹² is independently an alkyl group or an acidic group; and n² is an integer of 0 to 5.

Examples of the alkyl group include a methyl group, an ethyl group, a n-propyl group, an isopropyl group, a n-butyl group, a sec-butyl group, a tert-butyl group and a n-hexyl group, and these alkyl groups may further have a substituent such as a hydroxy group, a thiol group, an alkoxy group, an amino group or a nitrile group.

The linker represents a moiety that links X² and Z² in the formula (2) together, and examples thereof include alkylene moieties and an "(alkylene-oxygen atom)ₘ-alkylene linking moiety".

Examples of the alkylene moieties include a methylene moiety (-CH₂-), an ethylene moiety (-CH₂CH₂-), a propylene moiety (-CH₂CH₂CH₂-), a butylene moiety (-CH₂CH₂CH₂CH₂-) and an isopropylene moiety (-CH(CH₃)CH₂-), and being a propylene moiety is preferable.

Examples of the "(alkylene-oxygen atom)ₘ-alkylene linking moiety" (m is an integer of 1 to 5 and the alkylene and the alkylene linking moiety can be selected from the examples of the above alkylene moiety) include an ethoxyethyl moiety (-CH₂CH₂OCH₂CH₂-), a methoxyethyl moiety (-CH₂OCH₂CH₂-) and - CH₂CH₂O-CH₂CH₂O-CH₂CH₂-.

Examples of the acidic group include a sulfo group, a carboxy group, a phosphoric acid group and a hydroxy group, and being a sulfo group or a carboxy group is preferable. These acidic groups may be free acids, or may form salts.

It is preferable that n¹ is 1 to 4; being 1 or 2 is more preferable; and being 2 is especially preferable. In the case where n¹ is 2 or more, each X¹, each Y¹ and each Z¹ may be independently the same or different, respectively.

It is preferable that n² is 0 to 3; being 0 or 1 is more preferable; and being 0 is especially preferable. In the case where n² is 2 or more, each R¹² may be independently the same or different.

In the naphthoquinone-based compound represented by the formula (2), it is preferable that in the formula (2), X² is a sulfur atom; Y² is a propylene moiety; Z² is an acidic group; n¹ is 2; and n² is 0. That is, in the formula (2), it is preferable that no substituent is present on benzene rings in the naphthoquinone skeleton.

The naphthoquinone-based compound represented by the formula (2) may be used singly in one kind, or may be used in a combination of two or more kinds. In the case of using by combining two or more kinds, the two or more kinds can be used concurrently in any proportion.

### [Anthraquinone-based compound represented by the formula (3)]

In the formula (3), X³ is an oxygen atom or a sulfur atom; Y³ is a linker; Z³ is an acidic group; n³ is an integer of 3 to 8; each R¹³ is independently an alkyl group or an acidic group; and n⁴ is an integer of 0 to 5. In the formula (3), the linker, the acidic group and the alkyl group are the same as defined in the above formula (2), respectively.

It is preferable that X³ is an oxygen atom, and it is preferable that n³ is 3 to 6. Each X³, each Y³ and each Z³ may be independently the same or different, respectively.

In the anthraquinone-based compound represented by the formula (3), it is preferable that X³ is an oxygen atom; Y³ is a methylene moiety, a propylene moiety or -CH₂CH₂O-CH₂CH₂O-CH₂CH₂-; Z³ is a sulfo group, a carboxy group or a hydroxy group; n³ is 3, 4 or 6; and n⁴ is 0. More specifically, preferable are an anthraquinone-based compound in which in the formula (3), X³ is an oxygen atom, Y³ is a propylene moiety, Z³ is a sulfo group, n³ is 3, n⁴ is 0, and X³ are substituted on positions 1, 2 and 3 of the anthraquinone skeleton, respectively; an anthraquinone-based compound in which in the formula (3), X³ is an oxygen atom, Y³ is a propylene moiety, Z³ is a sulfo group, n³ is 6, n⁴ is 0, and X³ are substituted on positions 1, 2, 3, 5, 6 and 7 of the anthraquinone skeleton, respectively; an anthraquinone-based compound in which in the formula (3), X³ is an oxygen atom, Y³ is a methylene moiety, Z³ is a carboxy group, n³ is 6, n⁴ is 0, and X³ are substituted on positions 1, 2, 3, 5, 6 and 7 of the anthraquinone skeleton, respectively; and an anthraquinone-based compound in which in the formula (3), X³ is an oxygen atom, Y³ is a propylene moiety, Z³ is a sulfo group, n³ is 4, n⁴ is 0, and X³ are substituted on positions 1, 3, 5 and 7 of the anthraquinone skeleton, respectively.

The anthraquinone-based compound represented by the formula (3) may further have R¹³ as a substituent. It is preferable that R¹³ is an acidic group; and being a hydroxy group is more preferable. n⁴ suffices if the sum of the number of n³ and the number of n⁴ is set to be 8 or less; and it is preferable that n⁴ is 1 or 2; and it is especially preferable that n⁴ is 2.

More specifically, especially preferable is an anthraquinone-based compound in which in the formula (3), X³ is an oxygen atom, Y³ is -CH₂CH₂O-CH₂CH₂O-CH₂CH₂-, Z³ is a hydroxy group, n³ is 4, R¹³ is a hydroxy group, n⁴ is 0, and X³ are substituted on positions 1, 3, 5 and 7 of the anthraquinone skeleton, respectively, and R¹³ are substituted on positions 2 and 6 thereof, respectively. Such a compound is represented by the following formula (3').

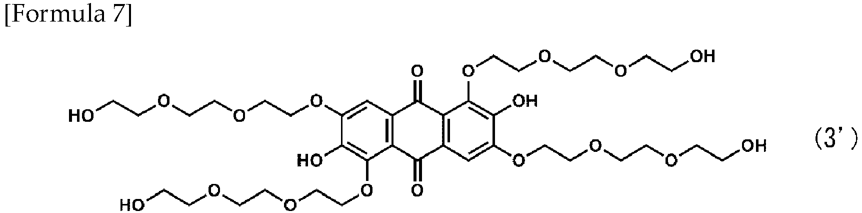

The anthraquinone-based compound represented by the formula (3) may be used singly in one kind, or may be used in a combination of two or more kinds. In the case of using by combining two or more kinds, the two or more kinds can be used concurrently in any proportion.

### <Active material>

An active material according to the present embodiment contains at least one heterocyclic compound represented by the formula (1), (2) or (3) or a salt thereof. By an aqueous electrolytic solution for a redox flow battery containing such an active material, the energy density and the cycle characteristic of the redox flow battery can be improved. The active material may contain any one of these heterocyclic compounds, or may contain two or more kinds thereof.

It is preferable that the active material is an active material for an aqueous electrolytic solution; and it is especially preferable that the active material is used for an electrolytic solution for a redox flow battery as an active material for an aqueous electrolytic solution. The active material contained in an electrolytic solution may be either one of a positive electrode active material and a negative electrode active material, but being a negative electrode active material is preferable.

### <Electrolytic solution>

An electrolytic solution according to the present embodiment contains the above-mentioned active material. For an active material according to the present embodiment, it is preferable, for enabling the improvement of the energy density and the cycle characteristic of a redox flow battery using an aqueous electrolytic solution, that the electrolytic solution is an electrolytic solution for a redox flow battery. As the active material in the electrolytic solution, the heterocyclic compounds represented by the formula (1), (2) and (3) may be contained singly in one kind, or may be contained in two or more kinds. In the case of using by combining two or more kinds of active material, the two or more kinds can be blended in any proportion. It is preferable that the content of the active material contained in the electrolytic solution is 1% by mass or higher and 99% by mass or lower; and being 3% by mass or higher and 70% by mass or lower is more preferable.

The electrolytic solution may further contain, other than the heterocyclic compounds represented by the formula (1), (2) and (3), optionally other compounds in the range of not impairing the effects of the present invention.

The electrolytic solution may contain water. As such a water, ion-exchange water, millipore water or the like can be used, and millipore water is preferable.

Although the content of water in the above electrolytic solution can optionally be set, being 1% by mass or higher and 99.9% by mass or lower is preferable; being 10% by mass or higher and 99% by mass or lower is more preferable; and being 75% by mass or higher and 95% by mass or lower is especially preferable.

In the case where the electrolytic solution contains, as the active material, a naphthoquinone-based compound represented by the formula (2) and/or an anthraquinone-based compound represented by the formula (3), that is, a quinone-based compound, it is preferable to contain a quinone-based compound having a solubility to water of higher than 0.07 mol/L; and it is more preferable that the solubility to water is 0.1 mol/L or higher and 5.0 mol/L or lower; being 0.15 mol/L or higher and 4.0 mol/L or lower is still more preferable; and being 0.18 mol/L or higher and 3.0 mol/L or lower is especially preferable.

The electrolytic solution may further contain an antifoaming agent. Examples of the antifoaming agent include alcohols such as methanol, ethanol and propanol, ketones such as acetone and methyl ethyl ketone, polyhydric alcohols such as ethylene glycol, diethylene glycol, propylene glycol and glycerol, and various kinds of commercially available antifoaming agents. Among these, it is preferable that the antifoaming agent is an alcohol; and being ethanol is especially preferable. The content of the antifoaming agent contained in the electrolytic solution is not especially limited, but it is preferable that the content is 0.1% by mass or higher and 10% by mass or lower with respect to the amount of water contained in the electrolytic solution; and being 0.5% by mass or higher and 8% by mass or lower is more preferable.

### <Redox flow battery>

A redox flow battery according to an aspect of the present invention includes the above-mentioned electrolytic solution. It is preferable that the redox flow battery further includes an electrode and a membrane.

The electrodes can be optionally selected and used as long as functioning as an electrode, but it is preferable to use, for example, a carbon felt, a carbon paper or a carbon cloth, and using a carbon felt is more preferable.

The membrane can be optionally selected and used as long as functioning as a membrane between electrodes, but it is preferable to use, for example, an ion-exchange membrane, a porous membrane or the like, and using an ion-exchange membrane is more preferable.

In the redox flow battery, the same electrolytic solution may be used for a positive electrode and a negative electrode, or different electrolytic solutions may be used. In the case of using different electrolytic solutions for a positive electrode and a negative electrode, the electrolytic solution according to the present embodiment and a counter electrolytic solution are used in combination.

In the redox flow battery, in the case of using different electrolytic solutions for a positive electrode and a negative electrode, it is preferable to use the electrolytic solution according to the present embodiment for the negative electrode side and a counter electrolytic solution for the positive electrode side, respectively, that is, to use the electrolytic solution according to the present embodiment as a negative electrode electrolytic solution and the counter electrolytic solution as a positive electrode electrolytic solution, respectively.

One of or both of the electrolytic solution and the counter electrolytic solution to be used for the redox flow battery may further contain an electrolyte. As the electrolyte, there can be used, for example, lithium hydroxide, sodium hydroxide, potassium hydroxide, lithium carbonate, sodium carbonate, potassium carbonate, ammonium carbonate, sodium hydrogencarbonate, potassium hydrogencarbonate, lithium chloride, sodium chloride, potassium chloride, ammonium chloride, sulfuric acid, acetic acid, formic acid or hydrochloric acid; and preferable are potassium hydroxide and sodium chloride, and especially preferable is sodium chloride.

The counter electrolytic solution is not especially limited as long as being one functioning as a positive electrode; there can be used, for example, potassium ferrocyanide, sodium ferrocyanide, ammonium ferrocyanide, ferrocene, TEMPO (2,2,6,6-tetramethylpiperidine-1-oxyl), lithium iodide, sodium iodide, potassium iodide, ammonium iodide or vanadium; and being a potassium ferrocyanide or sodium iodide aqueous solution is preferable, and being a sodium iodide aqueous solution is especially preferable.

The redox flow battery contains the electrolytic solution and optionally members including the counter electrolytic solution, the electrodes, the membrane, and the like, and in order to form the battery by using these members, as required, assembling members may further be used such as containers, sealants, screws and bipolar plates.

The electrolytic solution and the redox flow battery including the same according to the present embodiment, which contain the above-mentioned active material, has high energy density and also excellent cycle characteristic. In particular, a high energy density can be given to the redox flow battery using an aqueous electrolytic solution, and as compared with nonaqueous electrolytic solutions using organic solvents or the like as a solvent of the electrolytic solutions, the electrolytic solution is high in the safety, and is excellent in workability and the maintainability in fabrication of the redox flow battery, exchange of electrolytic solutions, and the like.

### Examples

Hereinafter, the present invention will be described in more detail by way of Examples, but the present invention is not limited thereto. Unless otherwise specified, room temperature is a temperature in the range of 20°C ± 5°C.

### [Synthesis Example 1]

17.6 parts of 1,2-phenylenediamine and 25 parts of 2,5-dihydroxy-1,4-benzoquinone were heated to reflux under stirring in 3,000 parts of water for 5 hours and 30 min, and thereafter cooled down to room temperature and stirred overnight. A black wet cake was obtained by filtration separation from the obtained suspension, and washed with water. The wet cake was vacuum dried at 80°C to thereby obtain 103.2 parts of a wet cake containing 0.163 mol of a compound represented by the following formula (4).

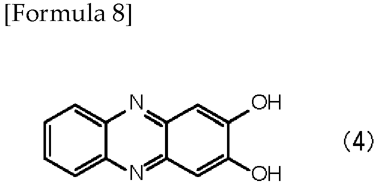

### [Example 1]

51.2 parts of the wet cake containing 0.0808 mol of the compound represented by the formula (4) obtained in Synthesis Example 1 and 36.6 parts of 1,8-diazabicyclo[5.4.0]undec-7-ene were dissolved in 410 parts of dimethylformamide; 30.2 parts of 1,3-propane sultone was added and then heated up to 120°C and stirred for 3 hours. Thereafter, the resultant was cooled down to room temperature and an excessive amount of a 25% sodium hydroxide aqueous solution was added and stirred for 30 min. The obtained reaction liquid was poured in 3.0 L of acetone, and a deposited solid was filtration separated to thereby obtain a wet cake. The wet cake was dissolved in 70 parts of water; and then, 10 parts of a 25% sodium hydroxide aqueous solution was added; thereafter, the resultant was poured in 1.5 L of ethanol; and a deposited solid was filtration separated to thereby obtain a red wet cake. The wet cake was vacuum dried at 80°C to thereby obtain 34.8 parts of a heterocyclic compound of the present invention represented by the following formula (1-1).

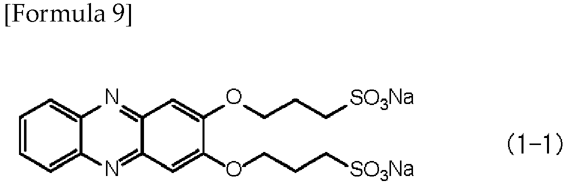

### [Synthesis Example 2]

20 parts of 2-nitro-1,4-phenylenediamine was dissolved in 230 parts of toluene to thereby obtain a solution. 9 parts of potassium carbonate was added to the solution and heated up to 60°C; 14.7 parts of acetic anhydride was added over 45 min with 60°C being held, and stirred for 1 hour. The resultant solution was cooled down to 25°C, and a solid deposited from the solution was filtration separated to thereby obtain 35 parts of a wet cake. The wet cake was dried in a hot-air dryer at 80°C to thereby obtain 23.9 parts of a compound represented by the following formula (5).

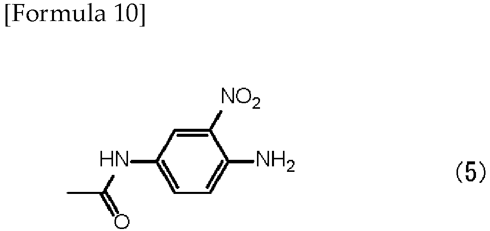

### [Synthesis Example 3]

23.9 parts of the compound represented by the formula (5) obtained in Synthesis Example 2 was stirred in 250 parts of ethanol for 30 min to thereby obtain a suspension. 0.5 part of a palladium/carbon (Pd: 10%, about 55% water-wetted product), manufactured by Tokyo Chemical Industry Co., Ltd., to the suspension; and the resultant mixture liquid was transferred to a pressure-resistant vessel. A hydrogen gas was injected in the pressure-resistant vessel and the pressure in the vessel was regulated at 0.95 MPa and the temperature in the vessel was raised up to 65°C. With the temperature in the vessel being held at 65°C, the hydrogen gas was injected so that the pressure became 0.95 MPa and the reaction was carried out for 4 hours. After the finish of the reaction, the pressure was returned to atmospheric pressure; and the resultant reaction liquid was subjected to filtration separation; and ethanol was distilled away under reduced pressure to thereby obtain 19.2 parts of a compound represented by the following formula (6).

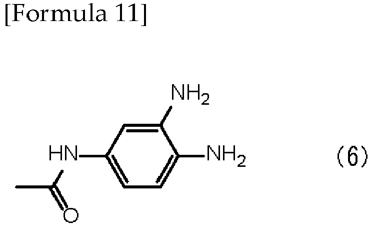

### [Synthesis Example 4]

The whole amount of the compound represented by the formula (6) obtained in Synthesis Example 3 and 19.7 parts of 2,5-dihydroxy-1,4-benzoquinone were, while being heated to reflux, stirred in 400 parts of water for 13 hours in total until the reaction came to the end. The obtained suspension was cooled down to room temperature and a deposited solid was filtration separated and then three times washed with ethanol to thereby obtain a purple wet cake. The wet cake was vacuum dried at 80°C to thereby obtain 44.8 parts of a wet cake containing 0.116 mol of a compound represented by the following formula (7).

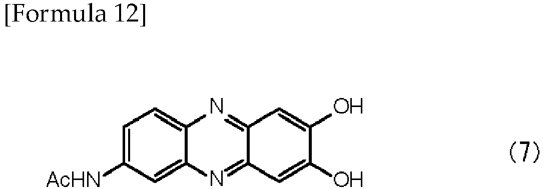

### [Example 2]

The whole amount of the wet cake containing the compound represented by the formula (7) obtained in Synthesis Example 4 and 59.6 parts of 1,8-diazabicyclo[5.4.0]undec-7-ene were dissolved in 530 parts of dimethylformamide; 48.2 parts of 1,3-propane sultone was added and then, the resultant was heated up to 120°C and stirred for 3 hours. Thereafter, the resultant was cooled down to room temperature and 50 parts of a 25% sodium hydroxide aqueous solution was added and stirred for 30 min. The obtained reaction liquid was poured in 2.0 L of acetone, and a deposited solid was filtration separated to thereby obtain a wet cake. The wet cake was dissolved in 300 parts of water; thereafter, the resultant was poured in 2.0 L of ethanol, and a deposited solid was filtration separated to thereby obtain a purple wet cake. The wet cake was vacuum dried at 70°C to thereby obtain 42.9 parts of a heterocyclic compound of the present invention represented by the following formula (1-2).

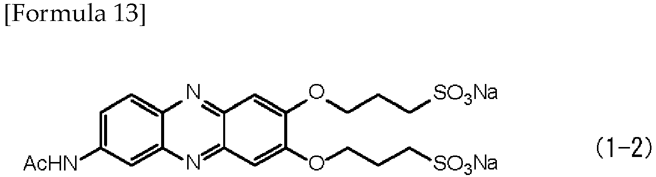

### [Example 3]

5.6 parts of the heterocyclic compound represented by the formula (1-2) obtained in Example 2 was dissolved in 50 parts of a 25% sodium hydroxide aqueous solution, and heated to reflux under stirring for 3 hours, and thereafter cooled down to room temperature and stirred overnight. Thereafter, the solvent was distilled away from the obtained reaction liquid by a rotary evaporator to thereby obtain a black solid. The solid was dissolved in 30 parts of water, and poured in 800 parts of ethanol, and then, a deposited solid was filtration separated and washed with ethanol. This operation was twice repeated; and a wet cake finally obtained was washed with acetone, and vacuum dried at 80°C to thereby obtain 3.7 parts of a heterocyclic compound of the present invention represented by the following formula (1-3).

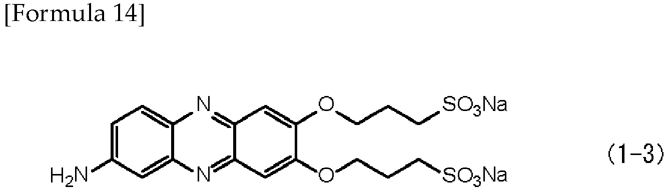

### [Example 4]

The heterocyclic compound represented by the formula (1-1) was dissolved in a sodium chloride (manufactured by Tokyo Chemical Industry Co., Ltd., purity: >99.5%) aqueous solution (1.0 mol/L) to attain 0.1 mol/L, to thereby fabricate a negative electrode electrolytic solution 1. On the other hand, sodium ferrocyanide (manufactured by Fujifilm Wako Pure Chemical Corp., content: 95% or higher) was dissolved in a sodium chloride (manufactured by Tokyo Chemical Industry Co., Ltd., purity: >99.5%) aqueous solution (1.0 mol/L) to attain 0.2 mol/L, to thereby fabricate a positive electrode electrolytic solution 1.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 1 and positive electrode electrolytic solution 1 were used as electrolytic solutions, to thereby fabricate a redox flow battery 1.

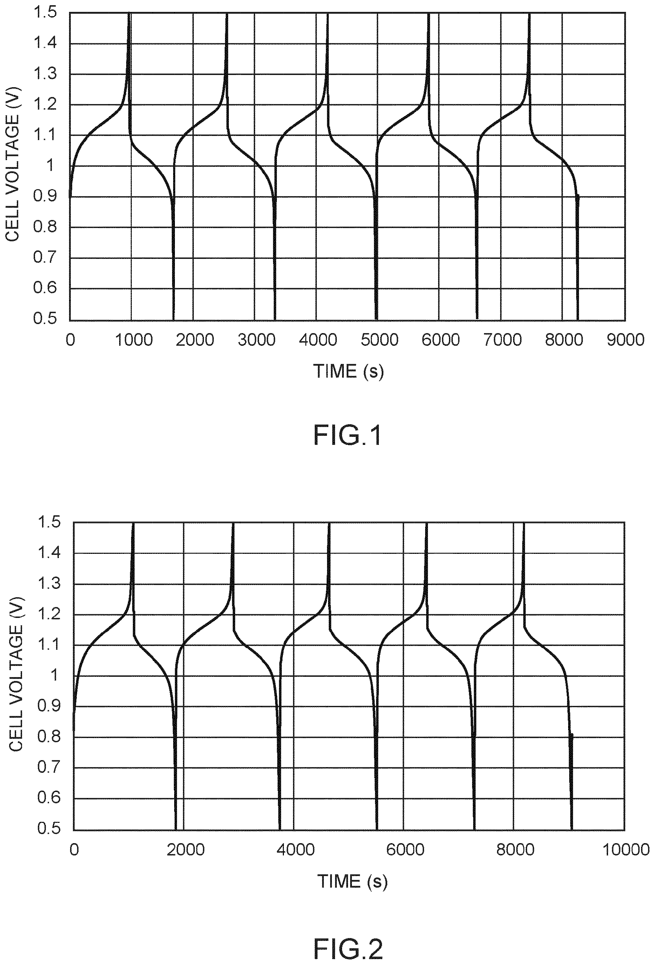

The positive electrode electrolytic solution 1 and the negative electrode electrolytic solution 1 of the redox flow battery 1 were circulated by peristaltic pumps piped and connected to outsides of the battery; and a test was carried out by using a multi-electrochemical measurement system (manufactured by Hokuto Denko Corp., HZ-Pro). The solution volumes of the positive electrode electrolytic solution 1 and the negative electrode electrolytic solution 1 were 20 ml and 6 ml, respectively; and a charge/discharge test was carried out at a constant current of 105 mA and with the upper limit voltage being set at 1.6 V and the lower limit voltage being set at 0.5 V. Fig. 1 shows a charge/discharge curve until the fifth cycle of the redox flow battery 1. In the fifth cycle, the coulomb efficiency was 92%; the voltage efficiency, 89%; and the energy density, 0.90 Wh/L, thus attaining a good cycle characteristic.

### [Example 5]

The heterocyclic compound represented by the formula (1-2) was dissolved in a sodium chloride (manufactured by Tokyo Chemical Industry Co., Ltd., purity: >99.5%) aqueous solution (1.0 mol/L) to attain 0.1 mol/L, to thereby fabricate a negative electrode electrolytic solution 2. On the other hand, sodium ferrocyanide (manufactured by Fujifilm Wako Pure Chemical Corp., content: 95% or higher) was dissolved in a sodium chloride (manufactured by Tokyo Chemical Industry Co., Ltd., purity: >99.5%) aqueous solution (1.0 mol/L) to attain 0.2 mol/L, to thereby fabricate a positive electrode electrolytic solution 2.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 2 and positive electrode electrolytic solution 2 were used as electrolytic solutions, to thereby fabricate a redox flow battery 2.

The positive electrode electrolytic solution 2 and the negative electrode electrolytic solution 2 of the redox flow battery 2 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 2 and the negative electrode electrolytic solution 2 were 20 ml and 6 ml, respectively; and the charge/discharge test was carried out at a constant current of 105 mA and with the upper limit voltage being set at 1.6 V and the lower limit voltage being set at 0.5 V. Fig. 2 shows a charge/discharge curve until the fifth cycle of the redox flow battery 2. In the fifth cycle, the coulomb efficiency was 96%; the voltage efficiency, 89%; and the energy density, 1.02 Wh/L, thus attaining a good cycle characteristic.

### [Example 6]

The heterocyclic compound represented by the formula (1-3) was dissolved in a sodium chloride (manufactured by Tokyo Chemical Industry Co., Ltd., purity: >99.5%) aqueous solution (1.0 mol/L) to attain 0.1 mol/L, to thereby fabricate a negative electrode electrolytic solution 3. On the other hand, sodium ferrocyanide (manufactured by Fujifilm Wako Pure Chemical Corp., content: 95% or higher) was dissolved in a sodium chloride (manufactured by Tokyo Chemical Industry Co., Ltd., purity: >99.5%) aqueous solution (1.0 mol/L) to attain 0.2 mol/L, to thereby fabricate a positive electrode electrolytic solution 3.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 3 and positive electrode electrolytic solution 3 were used as electrolytic solutions, to thereby fabricate a redox flow battery 3.

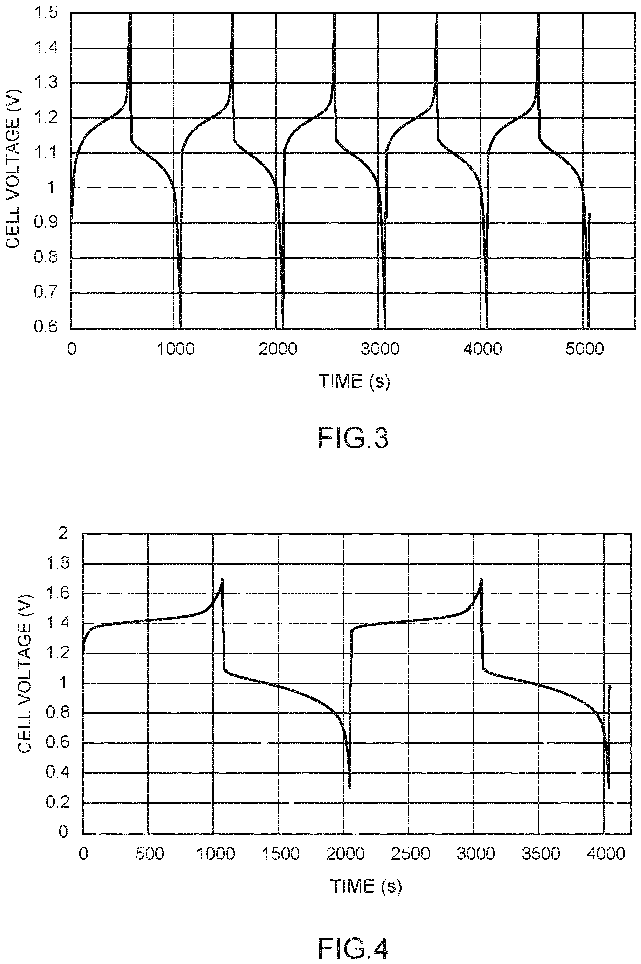

The positive electrode electrolytic solution 3 and the negative electrode electrolytic solution 3 of the redox flow battery 3 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 3 and the negative electrode electrolytic solution 3 were 20 ml and 6 ml, respectively; and the charge/discharge test was carried out at a constant current of 105 mA and with the upper limit voltage being set at 1.5 V and the lower limit voltage being set at 0.6 V. Fig. 3 shows a charge/discharge curve until the fifth cycle of the redox flow battery 3. In the fifth cycle, the coulomb efficiency was 98%; the voltage efficiency, 88%; and the energy density, 0.57 Wh/L, thus attaining a good cycle characteristic.

**[Table 1]**

| Example | Battery | Active material | Average discharge voltage (V) | Coulomb efficiency (%) | Voltage efficiency (%) | Energy density (Wh/L) |
|---|---|---|---|---|---|---|
| 4 | redox flow battery 1 | formula (1-1) | 1.03 | 92 | 89 | 0.90 |
| 5 | redox flow battery 2 | formula (1-2) | 1.05 | 96 | 89 | 1.02 |
| 6 | redox flow battery 3 | formula (1-3) | 1.05 | 98 | 88 | 0.57 |

### [Examples 7 and 8]

2.5 g of the heterocyclic compound represented by the formula (1-1) obtained in Example 1 was weighed in a 10-mL measuring flask and diluted with an aqueous solution containing an antifoaming agent so the whole as to become 10 ml. 6 ml of the obtained electrolytic solution was transferred in a 20 ml-volume screw vial (outer diameter: 27 mm, height: 55 mm, SV-20, manufactured by Nichiden-Rika Glass Co., Ltd.). The screw vial containing the electrolytic solution was capped and shaken for 30 s at a pace of 110 times/30 s so the vertical motion width as to become 15 cm, and allowed to stand still for 5 min; thereafter, there was measured the minimum value of the height of air bubbles from the liquid level on the wall surface. Whereas in the case (Example 8) of adding no antifoaming agent indicated in Table 2, air bubbles remained, by adding an antifoaming agent, air bubbles disappeared. Here, the hyphen "-" in Table 2 indicates that there were no air bubbles and the height of air bubbles could not be measured.

**[Table 2]**

| Example | Antifoaming agent | Height of air bubbles |
|---|---|---|
| 7 | ethanol (6% by mass to water) | - |
| 8 | no add itive | 11 mm |

### [Example 9]

The heterocyclic compound represented by the formula (1-1) was dissolved in a 6-wt% ethanol aqueous solution to attain 0.5 mol/L to thereby fabricate a negative electrode electrolytic solution 4. On the other hand, sodium iodide (manufactured by Junsei Chemical Co., Ltd., first grade) was dissolved in a 6-wt% ethanol aqueous solution to attain 2.0 mol/L to thereby fabricate a positive electrode electrolytic solution 4.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 4 and positive electrode electrolytic solution 4 were used as electrolytic solutions, to thereby fabricate a redox flow battery 4.

The positive electrode electrolytic solution 4 and the negative electrode electrolytic solution 4 of the redox flow battery 4 obtained above were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 4 and the negative electrode electrolytic solution 4 were 6 ml, respectively; and the charge/discharge test was carried out at a constant current of 420 mA and with the upper limit voltage being set at 1.7 V and the lower limit voltage being set at 0.3 V. Fig. 4 shows a charge/discharge curve until the second cycle of the redox flow battery 4. In the second cycle, the coulomb efficiency was 97%; the voltage efficiency, 66%; and the energy density, 8.92 Wh/L, thus attaining a good cycle characteristic.

**[Table 3]**

| Example | Battery | Antifoaming agent | Average discharge voltage (V) | Coulomb efficiency (%) | Voltage efficiency (%) | Energy density (Wh/L) |
|---|---|---|---|---|---|---|
| 9 | redox flow battery 4 | 6-mass% ethanol | 0.94 | 97 | 66 | 8.92 |

### [Example 10]

50 parts of dimethylformamide was added to 2.12 parts of the heterocyclic compound represented by the formula (1-1) obtained in Example 1, 8.31 parts of potassium carbonate and 0.17 part of potassium iodide, and 3.51 parts of bromoacetic acid was dropwise added thereto and stirred at room temperature for 20 hours. The reaction liquid was poured in 500 mL of acetone, and a red wet cake was obtained by filtration separation, and was then washed with a large amount of acetone. The wet cake was dissolved in 40 parts of water, and a 2N hydrochloric acid was dropped to regulate the pH at 1.0. A black wet cake was obtained again by filtration separation, and was then washed with water and methanol, and vacuum dried at 80°C to thereby obtain 2.2 parts of a heterocyclic compound of the present invention represented by the following formula (1-4).

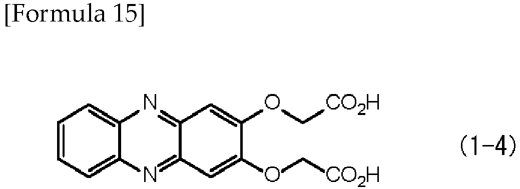

### [Synthesis Example 5]

2.52 parts of 3,4-diaminobenzoic acid and 2.56 parts of 2,5-dihydroxy-1,4-benzoquinone were heated to reflux under stirring in 110 parts of water for 3 hours, and thereafter cooled down to room temperature and stirred overnight. A red wet cake was obtained by filtration separation from the obtained suspension, and washed with acetone and water. The wet cake was vacuum dried at 80°C to thereby obtain 4.3 parts of a compound represented by the following formula (8).

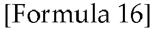

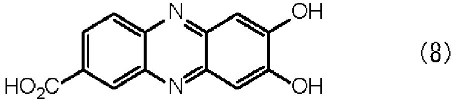

### [Example 11]

2.58 parts of the compound represented by the formula (8) obtained in Synthesis Example 5 and 4.90 parts of 1,8-diazabicyclo[5.4.0]undec-7-ene were dissolved in 50 parts of dimethylformamide; 3.81 parts of 1,3-propane sultone was added and then, the resultant was heated up to 120°C and stirred for 1 hour. Thereafter, 2.28 parts of 1,8-diazabicyclo[5.4.0]undec-7-ene and 1.82 parts of 1,3-propane sultone were further added, and stirred at 120°C for 6 hours. The reaction liquid was cooled down to room temperature; 9.6 parts of a 25% sodium hydroxide aqueous solution was dropwise added and stirred for 1 hour; and the resultant was poured in 500 mL of acetone. A red wet cake was obtained by filtration separation, and washed with acetone. The wet cake was dissolved in 80 parts of water, and poured in 1.0 L of ethanol, and then, a deposited solid was filtered off and washed with ethanol to thereby obtain a brown wet cake, which was then further vacuum dried at 80°C to thereby obtain 2.7 parts of a heterocyclic compound of the present invention represented by the following formula (1-5).

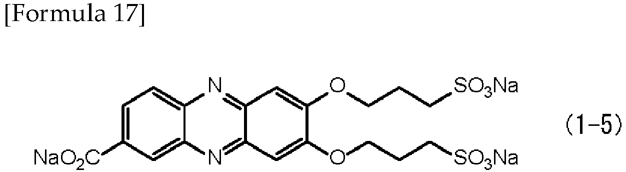

### [Example 12]

The heterocyclic compound represented by the formula (1-4) was dissolved in a sodium hydroxide (manufactured by Kokusan Chemical Co., Ltd., content: ≥ 97.0%) aqueous solution (1.0 mol/L) to attain 0.1 mol/L to thereby fabricate a negative electrode electrolytic solution 5. On the other hand, sodium ferrocyanide (manufactured by Fujifilm Wako Pure Chemical Corp., content: 95% or higher) was dissolved in a sodium hydroxide (manufactured by Kokusan Chemical Co., Ltd., content: ≥ 97.0%) aqueous solution (1.0 mol/L) to attain 0.2 mol/L to thereby fabricate a positive electrode electrolytic solution 5.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 5 and positive electrode electrolytic solution 5 were used as electrolytic solutions, to thereby fabricate a redox flow battery 5.

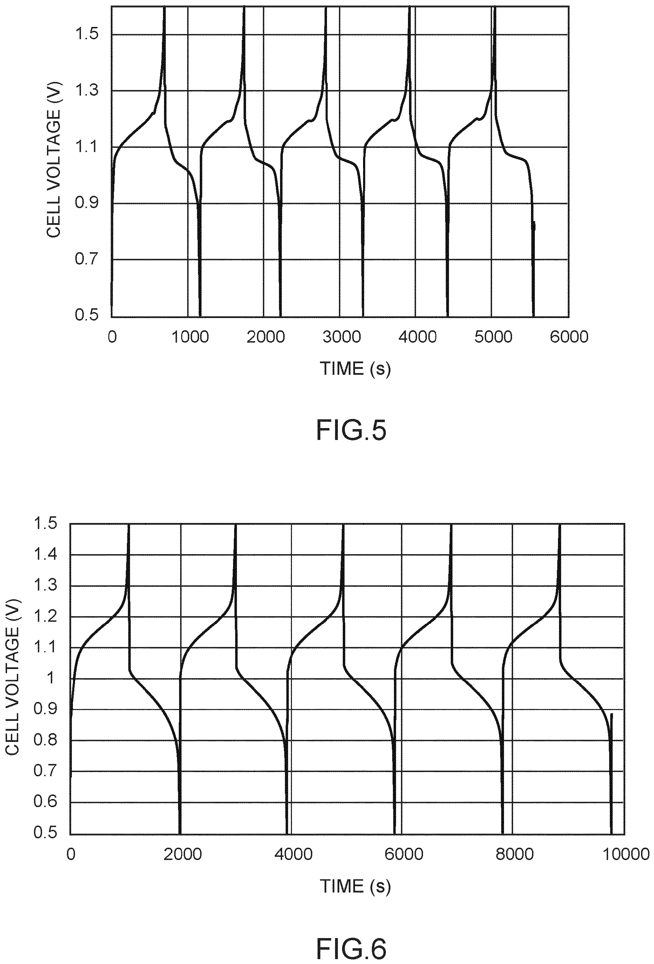

The positive electrode electrolytic solution 5 and the negative electrode electrolytic solution 5 of the redox flow battery 5 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 5 and the negative electrode electrolytic solution 5 were 20 ml and 6 ml, respectively; and the charge/discharge test was carried out at a constant current of 105 mA and with the upper limit voltage being set at 1.6 V and the lower limit voltage being set at 0.5 V. Fig. 5 shows a charge/discharge curve until the fifth cycle of the redox flow battery 5. In the fifth cycle, the coulomb efficiency was 81%; the voltage efficiency, 88%; and the energy density, 0.59 Wh/L, thus attaining a good cycle characteristic.

### [Example 13]

The heterocyclic compound represented by the formula (1-5) was dissolved in a sodium chloride (manufactured by Tokyo Chemical Industry Co., Ltd., purity: >99.5%) aqueous solution (1.0 mol/L) to attain 0.1 mol/L, to thereby fabricate a negative electrode electrolytic solution 6. On the other hand, sodium ferrocyanide (manufactured by Fujifilm Wako Pure Chemical Corp., content: 95% or higher) was dissolved in a sodium chloride (manufactured by Tokyo Chemical Industry Co., Ltd., purity: >99.5%) aqueous solution (1.0 mol/L) to attain 0.2 mol/L, to thereby fabricate a positive electrode electrolytic solution 6.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 6 and positive electrode electrolytic solution 6 were used as electrolytic solutions, to thereby fabricate a redox flow battery 6.

The positive electrode electrolytic solution 6 and the negative electrode electrolytic solution 6 of the redox flow battery 6 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 6 and the negative electrode electrolytic solution 6 were 20 ml and 6 ml, respectively; and the charge/discharge test was carried out at a constant current of 105 mA and with the upper limit voltage being set at 1.5 V and the lower limit voltage being set at 0.5 V. Fig. 6 shows a charge/discharge curve until the fifth cycle of the redox flow battery 6. In the fifth cycle, the coulomb efficiency was 89%; the voltage efficiency, 81%; and the energy density, 0.98 Wh/L, thus attaining a good cycle characteristic.

**[Table 4]**

| Example | Battery | Active material | Average discharge voltage (V) | Coulomb efficiency (%) | Voltage efficiency (%) | Energy density (Wh/L) |
|---|---|---|---|---|---|---|
| 12 | redox flow battery 5 | formula (1-4) | 1.06 | 81 | 88 | 0.59 |
| 13 | redox flow battery 6 | formula (1-5) | 0.95 | 89 | 81 | 0.98 |

### [Example 14]

3.03 parts of the compound represented by the formula (4) obtained in Synthesis Example 1 and 1.60 parts of 1,8-diazabicyclo[5.4.0]undec-7-ene were dissolved in 100 parts of dimethylformamide; 1.28 parts of 1,3-propane sultone was added and then, the resultant was heated up to 50°C and stirred for 2 hours. The reaction liquid was cooled down to room temperature; 8.0 parts of a 25% sodium hydroxide aqueous solution was dropwise added and stirred for 1 hour; and thereafter, the resultant was poured in 500 mL of acetone. A brown wet cake was obtained by filtration separation, and washed with acetone. The wet cake was dissolved in 50 parts of water, and poured in 500 mL of 2-propanol, and then, a deposited solid was filtered off and washed with ethanol to thereby obtain a brown wet cake, which was then vacuum dried at 80°C to thereby obtain 1.4 parts of a brown powder containing a heterocyclic compound of the present invention represented by the following formula (1-6).

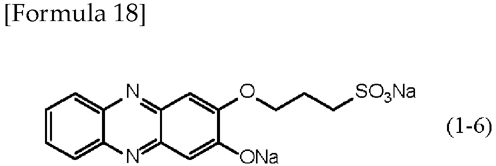

### [Example 15]

The heterocyclic compound represented by the formula (1-6) was dissolved in a sodium hydroxide (manufactured by Kokusan Chemical Co., Ltd., content: ≥ 97.0%) aqueous solution (1.0 mol/L) to attain 0.1 mol/L to thereby fabricate a negative electrode electrolytic solution 7. On the other hand, sodium ferrocyanide (manufactured by Fujifilm Wako Pure Chemical Corp., content: 95% or higher) was dissolved in a sodium hydroxide (manufactured by Kokusan Chemical Co., Ltd., content: ≥ 97.0%) aqueous solution (1.0 mol/L) to attain 0.2 mol/L to thereby fabricate a positive electrode electrolytic solution 7.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The above negative electrode electrolytic solution 7 and positive electrode electrolytic solution 7 were used as electrolytic solutions, to thereby fabricate a redox flow battery 7.

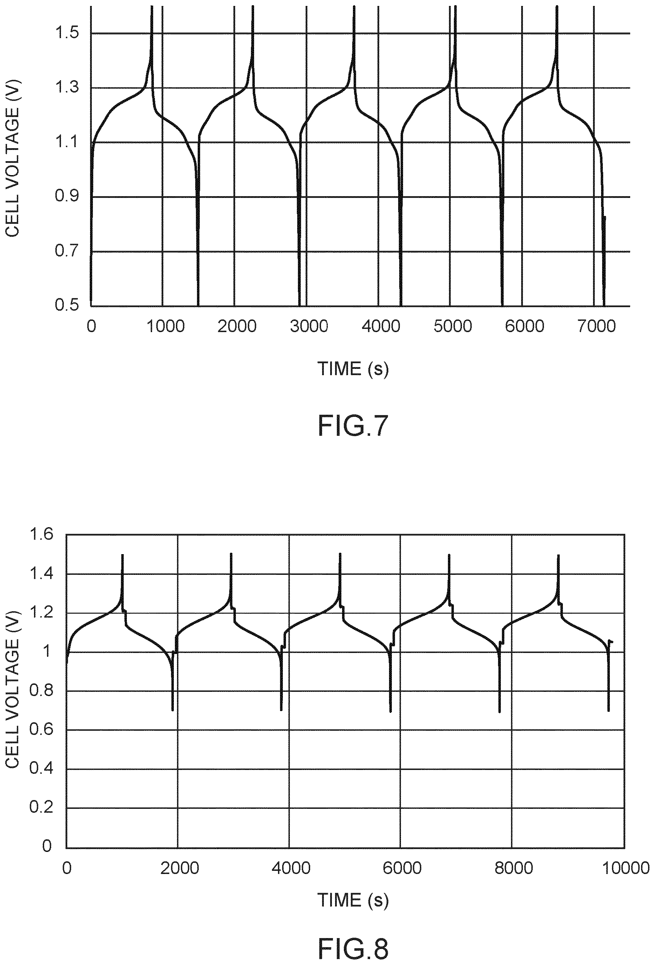

The positive electrode electrolytic solution 7 and the negative electrode electrolytic solution 7 of the redox flow battery 7 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 7 and the negative electrode electrolytic solution 7 were 20 ml and 6 ml, respectively; and the charge/discharge test was carried out at a constant current of 105 mA and with the upper limit voltage being set at 1.6 V and the lower limit voltage being set at 0.5 V. Fig. 7 shows a charge/discharge curve until the fifth cycle. In the fifth cycle, the coulomb efficiency was 86%; the voltage efficiency, 91%; and the energy density, 0.83 Wh/L, thus attaining a good cycle characteristic.

**[Table 5]**

| Example | Battery | Active material | Average discharge voltage (V) | Coulomb efficiency (%) | Voltage efficiency (%) | Energy density (Wh/L) |
|---|---|---|---|---|---|---|
| 15 | redox flow battery 7 | formula (1-6) | 1.15 | 86 | 91 | 0.83 |

As indicated in the above Tables 1, 3, 4 and 5 and Figs. 1 to 7, it is clear that the redox flow batteries 1 to 7 fabricated in Examples 4 to 6, 9, 12, 13 and 15 had a high average discharge voltage (V) and a high energy density, and had a good cycle characteristic. Further it can be confirmed that in Example 7 in which ethanol was added as an antifoaming agent in the electrolytic solution, no generation of air bubbles occurred and the electrolytic solution was excellent in handleability.

### [Synthesis Example 6]

20 parts of gallic acid was charged in 60 parts of sulfuric acid and stirred, and stirred at 105°C for 3 hours. The reaction liquid was cooled down to 50°C and poured in 150 parts of ice water and the obtained suspension was stirred for 1 hour. After the stirring, the suspension was subjected to filtration separation to thereby obtain a wet cake. The wet cake was added to 150 parts of cold water and stirred for 30 min; thereafter, the resultant suspension was subjected to filtration separation to thereby obtain a wet cake. The wet cake was little by little added in 150 parts of a 5% sodium hydrogencarbonate aqueous solution, and after stirring for 1 hour, the suspension was subjected to filtration separation to thereby obtain a wet cake. The wet cake was dried in a hot-air dryer at 80°C to thereby obtain 12.4 parts of a compound represented by the following formula (9).

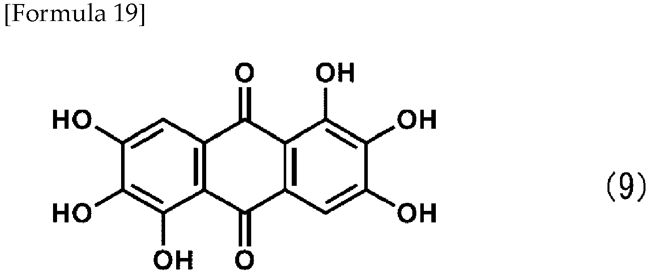

### [Example 16]

5 parts of the compound represented by the formula (9) was charged and stirred in 45 parts of water; the pH of the solution was regulated at 9.5 to 10.0 with a 25% sodium hydroxide aqueous solution; and the resultant was heated up to 60°C. 20 parts of propane sultone was dropped in the solution over 2 hours, and after the finish of the dropping, the resultant was stirred at 60°C for 4 hours. During this, the pH of the solution was held at 10.0 to 10.5 with a 25% sodium hydroxide aqueous solution. 500 parts of methanol was added to the obtained reaction liquid, and stirred at 20 to 25°C for 2 hours. After the stirring, a deposited solid was filtration separated to thereby obtain 40.8 parts of a wet cake. The wet cake was dried in a hot-air dryer at 80°C to thereby obtain 9.2 parts of a heterocyclic compound of the present invention represented by the following formula (3-1).

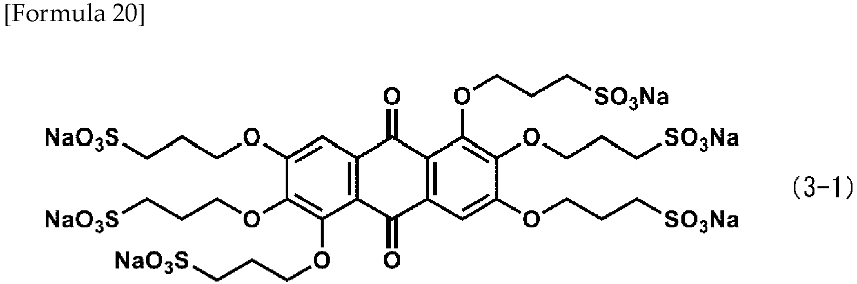

### [Example 17]

The same operation as in Example 15 was carried out, except for using 20.5 parts of bromoacetic acid in place of 20 parts of propane sultone and using potassium hydroxide in place of sodium hydroxide, to thereby obtain 7.9 parts of a heterocyclic compound of the present invention represented by the following formula (3-2).

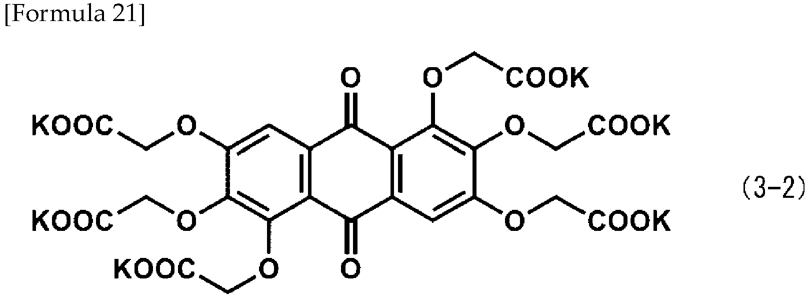

### [Synthesis Example 7]

10 parts of benzoic acid was charged in 60 parts of sulfuric acid and stirred, and heated up to 120°C. 13.9 parts of gallic acid was little by little added to the obtained solution and stirred for 7 hours. The reaction liquid was cooled down to 50°C, and added to 150 parts of ice water; and the obtained suspension was stirred for 1 hour. After the stirring, the suspension was subjected to filtration separation to thereby obtain a wet cake. The wet cake was added to 150 parts of cold water, and stirred for 30 min; and thereafter, the resultant suspension was subjected to filtration separation to thereby obtain a wet cake. The wet cake was little by little added in 150 parts of 5% sodium hydrogencarbonate aqueous solution; and after the stirring for 1 hour, the resultant suspension was subjected to filtration separation to thereby obtain a wet cake. The wet cake was dried in a hot-air dryer at 80°C to thereby obtain 13.1 parts of a compound represented by the following formula (10).

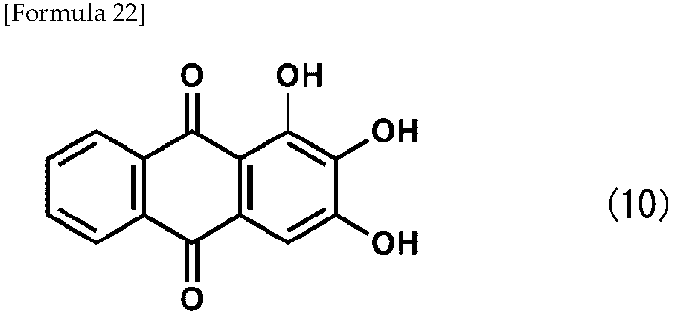

### [Example 18]

5 parts of the compound represented by the formula (10) was charged and stirred in 45 parts of water; the pH of the solution was regulated at 9.5 to 10.0 with a 25% sodium hydroxide aqueous solution; and the resultant was heated up to 60°C. 11.9 parts of propane sultone was dropped in the solution over 2 hours, and after the finish of the dropping, the resultant was stirred at 60°C for 4 hours. During this, the pH of the solution was held at 10.0 to 10.5 with a 25% sodium hydroxide aqueous solution. 500 parts of methanol was added to the obtained reaction liquid, and stirred at 20 to 25°C for 2 hours. A deposited solid was filtration separated to thereby obtain 30.7 parts of a wet cake. The wet cake was dried in a hot-air dryer at 80°C to thereby obtain 10.8 parts of a heterocyclic compound of the present invention represented by the following formula (3-3).

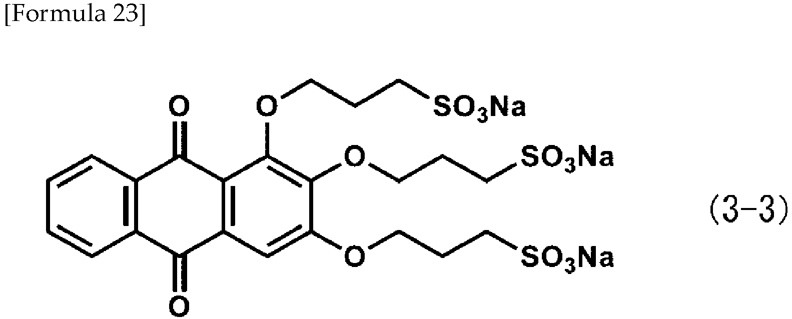

### [Example 19]

10 parts of 2,3-dichloro-1,4-naphthoquinone was dissolved in 200 parts of N,N-dimethylformamide; and 17 parts of 3-mercapto-1-propanesulfonic acid and 18 parts of potassium carbonate were added, and the resultant was stirred at room temperature for 12 hours. An obtained deposited solid was filtration separated and washed with ethanol to thereby obtain a red solid. The obtained solid was dissolved in water and a 1N hydrochloric acid aqueous solution was added until no generation of carbon dioxide occurred. Isopropanol was added to the obtained reaction liquid and then, a deposited solid was filtration separated to thereby obtain a wet cake. The wet cake was dried in a hot-air dryer at 80°C to thereby obtain 23 parts of a heterocyclic compound of the present invention represented by the following formula (2-1).

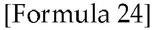

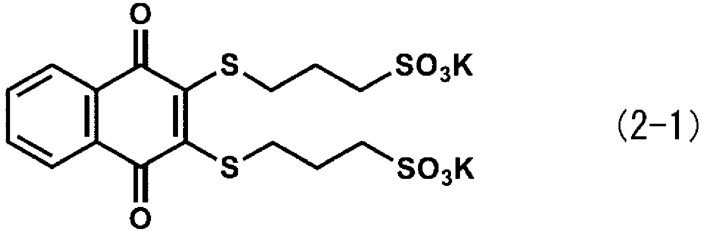

### [Synthesis Example 8]

120 parts of concentrated sulfuric acid was heated up to 120°C and stirred; and 40 parts of 3,5-dihydroxybenzoic acid was added thereto and stirred for 5 hours. The reaction liquid was cooled down to room temperature, and added to 500 parts of ice; and a 25% sodium hydroxide aqueous solution was added dropwise under stirring of the obtained suspension to regulate the pH at 7.5. The obtained suspension was subjected to filtration separation and washed with ice water to thereby obtain a wet cake containing a compound represented by the following formula (11).

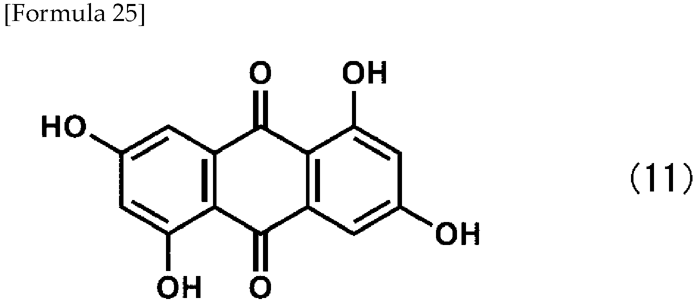

### [Example 20]

The wet cake containing the compound represented by the formula (11) obtained in Synthesis Example 8 was dissolved in 240 mL of water, and 3/4 of the whole amount thereof was transferred to a 1-L beaker. A 25% sodium hydroxide aqueous solution was added to the resultant aqueous solution to regulate the pH at 10.0 to 10.2, and the resultant was heated up to 60°C. 7 equivalents in total of propane sultone were added dropwise to the solution, and stirred at 60°C for 6 hours. During this, the pH of the solution was held at 10.0 to 10.2 with a 25% sodium hydroxide aqueous solution. The obtained reaction liquid was poured in 800 mL of methanol, and then, a deposited solid was filtration separated to thereby obtain a wet cake. The wet cake was dissolved in 100 parts of a 25% sodium hydroxide aqueous solution, and poured in 800 mL of methanol, and then, a deposited solid was filtration separated to thereby obtain a wet cake. This operation was carried out twice in total. The obtained wet cake was dissolved in 100 parts of water; and the pH was regulated at 10.0 by using a 35% hydrochloric acid and the resultant was heated up to 60°C. 4 equivalents of propane sultone were added dropwise thereto and stirred at 60°C for 5 hours. The obtained reaction liquid was poured in 800 mL of methanol and then, a deposited solid was filtration separated to thereby obtain a wet cake. The wet cake was dried in a hot-air dryer at 80°C to thereby obtain 56.6 parts of a heterocyclic compound of the present invention represented by the following formula (3-4).

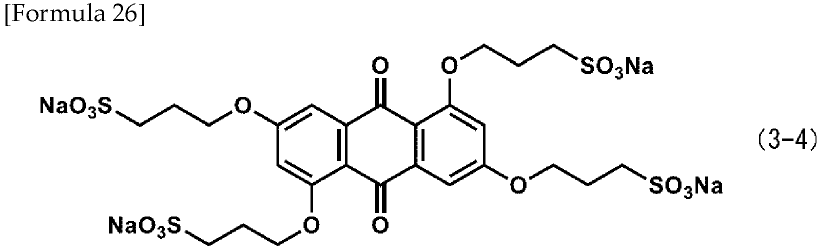

### [Example 21]

5 parts of the compound represented by the above formula (9), 9.1 parts of potassium carbonate and 2.7 parts of potassium iodide were charged and stirred in 45 parts of acetonitrile, and heated up to 80°C. 16.4 parts of ethylene glycol mono-2-chloroethyl ether was dropped in the resultant solution over 30 min, and after the finish of the dropping, the resultant was stirred at 80°C for 36 hours. The solvent of the obtained reaction liquid was distilled away under reduced pressure; 20 parts of water was added; and a brown fraction was taken by using a SEP-PACK (WATO043345), manufactured by Waters Corp. This aqueous solution was dried in a hot-air dryer at 80°C to thereby obtain 8.4 parts of a heterocyclic compound of the present invention represented by the following formula (3-5).

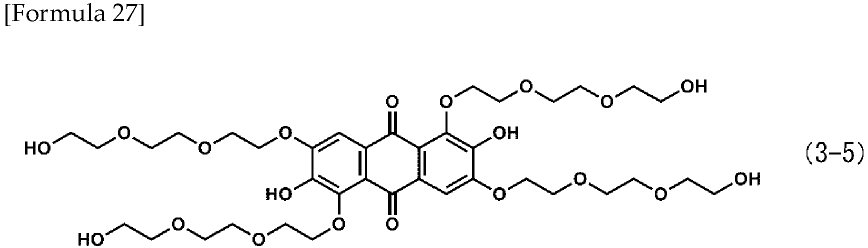

The solubility to water of each heterocyclic compound (active material) obtained in Examples 16 to 21 was calculated from the absorbance. The measurement of the absorbance used an ultraviolet-visible spectrometer (UV-1700, manufactured by Shimadzu Corp.). Solutions of known concentrations of a sample were prepared by using a standard buffer solution (manufactured by Fujifilm Wako Pure Chemical Corp., a neutral phosphate salt pH standard solution, pH: 6.86 (25°C)), and the absorbances at the maximum absorption wavelength in the wavelength region of 300 nm to 550 nm were measured by the ultraviolet-visible spectrometer. A calibration curve was fabricated from the obtained absorbances and the concentrations. Then, a saturated solution of the sample was prepared by using water and was diluted by using the standard buffer solution. The absorbance at the maximum absorption wavelength was measured and the solubility (%) was calculated from the calibration curve. The density of the solution was assumed to be 1.0 g/cm³ and the solubility (mol/L) was calculated. The results are shown in Table 6. Here, solubilities (mol/L) of conventionally well-known anthraquinone-based active materials are shown in Table 6 for reference.

**[Table 6]**

| Active material | Solubility (%) | Solubility (mol/L) |
|---|---|---|
| 9,10-anthraquinone-2-sulfonic acid (Na) | 0.7 | 0.02 |
| 9,1 0-anthraquinone-1 ,5-disulfonic acid (Na) | 2.7 | 0.07 |
| 9,10-anthraquinone-1,8-disulfonic acid (K) | 0.2 | 0.004 |
| formula (3-1) | 47.5 | 0.41 |
| formula (3-2) | 17.7 | 0.2 |
| formula (3-3) | 35.3 | 0.51 |
| formula (2-1) | 36.6 | 0.67 |
| formula (3-4) | 47.7 | 0.56 |
| formula (3-5) | 41.8 | 0.5 |

### [Example 22]

The heterocyclic compound represented by the formula (3-1) was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 8. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 8.

An ion-exchange membrane (manufactured by Sigma-Aldrich Japan, Nafion(R) NRE-212) was used as a membrane, and carbon felts (manufactured by Toyobo Co., Ltd., AAF304ZS, 10 mm × 50 mm × 4 mm) were used as electrodes. The carbon felts were each put in the hole of 10 mm × 50 mm of a silicon-made gasket (thickness: 3 mm), and these were assembled so as to make a current collecting plate/an electrode/a membrane/an electrode/a current collecting plate in this order. The fabricated negative electrode electrolytic solution 8 and positive electrode electrolytic solution 8 were used as electrolytic solutions, to thereby fabricate a redox flow battery 8.

The positive electrode electrolytic solution 8 and the negative electrode electrolytic solution 8 of the redox flow battery 8 were circulated by peristaltic pumps piped and connected to outsides of the battery; and the test was carried out by using the multi-electrochemical measurement system (manufactured by Hokuto Denko Corp., HZ-Pro). The solution volumes of the positive electrode electrolytic solution 7 and the negative electrode electrolytic solution 7 were 20 ml and 6 ml, respectively; and the charge/discharge test was carried out at a constant current of 105 mA and with the upper limit voltage being set at 1.5 V and the lower limit voltage being set at 0.7 V. Fig. 8 shows a charge/discharge curve until the fifth cycle of the redox flow battery 7. In the fifth cycle, the coulomb efficiency was 84%; the voltage efficiency, 92%; and the energy density, 1.04 Wh/L, thus attaining a good cycle characteristic.

### [Example 23]

The heterocyclic compound represented by the formula (3-2) was dissolved in a potassium chloride (manufactured by Junsei Chemical Co., Ltd., special grade) aqueous solution (1.0 mol/L) to attain 0.1 mol/L to thereby fabricate a negative electrode electrolytic solution 9. On the other hand, potassium iodide (manufactured by Junsei Chemical Co., Ltd., special grade) was dissolved in a potassium chloride (manufactured by Junsei Chemical Co., Ltd., special grade) aqueous solution (1.0 mol/L) to attain 0.2 mol/L to thereby fabricate a positive electrode electrolytic solution 9.

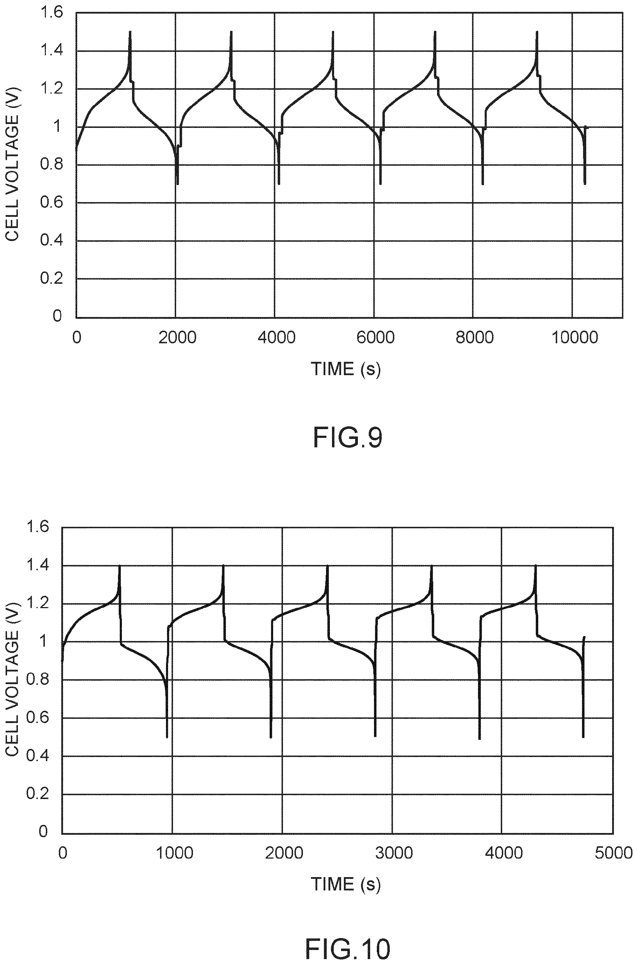

A redox flow battery 9 was fabricated as in Example 22, except for altering the negative electrode electrolytic solution 8 and the positive electrode electrolytic solution 8 in Example 22 to the negative electrode electrolytic solution 9 and the positive electrode electrolytic solution 9, respectively. By using the redox flow battery 9, the charge/discharge test was carried out by the same operation as in Example 22. The solution volumes of the positive electrode electrolytic solution 9 and the negative electrode electrolytic solution 9 were 20 ml and 6 ml, respectively; and the charge/discharge test was carried out at a constant current of 105 mA and with the upper limit voltage being set at 1.5 V and the lower limit voltage being set at 0.7 V. Fig. 9 shows a charge/discharge curve until the fifth cycle of the redox flow battery 8. In the fifth cycle, the coulomb efficiency was 87%; the voltage efficiency, 88%; and the energy density, 1.08 Wh/L.

### [Example 24]

The heterocyclic compound represented by the formula (3-3) was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 10. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 10.

A redox flow battery 10 was fabricated as in Example 22, except for altering the negative electrode electrolytic solution 8 and the positive electrode electrolytic solution 8 in Example 22 to the negative electrode electrolytic solution 10 and the positive electrode electrolytic solution 10, respectively. By using the redox flow battery 10, the charge/discharge test was carried out by the same operation as in Example 22. The solution volumes of the positive electrode electrolytic solution 10 and the negative electrode electrolytic solution 10 were 20 ml and 6 ml, respectively; and the charge/discharge test was carried out at a constant current of 105 mA and with the upper limit voltage being set at 1.4 V and the lower limit voltage being set at 0.5 V. Fig. 10 shows a charge/discharge curve until the fifth cycle of the redox flow battery 9. In the fifth cycle, the coulomb efficiency was 84%; the voltage efficiency, 82%; and the energy density, 0.46 Wh/L.

### [Example 25]

The heterocyclic compound represented by the formula (2-1) was dissolved in a potassium hydroxide (manufactured by Junsei Chemical Co., Ltd., special grade) aqueous solution (1.0 mol/L) to attain 0.1 mol/L to thereby fabricate a negative electrode electrolytic solution 11. On the other hand, potassium ferrocyanide (manufactured by Kanto Chemical Co., Inc., special grade) was dissolved in a potassium hydroxide (manufactured by Junsei Chemical Co., Ltd.) aqueous solution (1.0 mol/L) to attain 0.2 mol/L to thereby fabricate a positive electrode electrolytic solution 11.

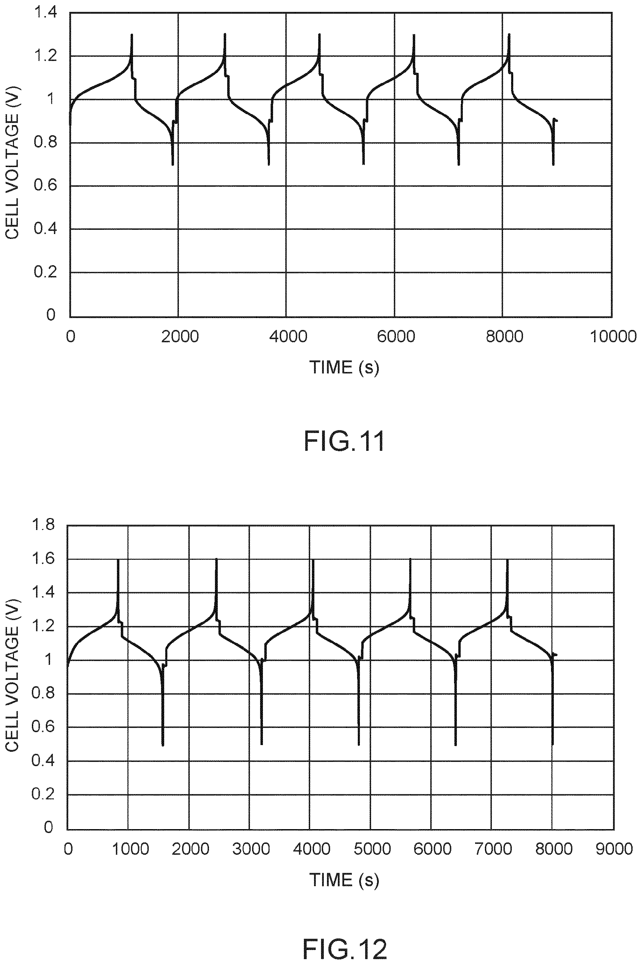

A redox flow battery 11 was fabricated as in Example 22, except for altering the negative electrode electrolytic solution 8 and the positive electrode electrolytic solution 8 in Example 22 to the negative electrode electrolytic solution 11 and the positive electrode electrolytic solution 11, respectively. By using the redox flow battery 11, the charge/discharge test was carried out by the same operation as in Example 22. The solution volumes of the positive electrode electrolytic solution 11 and the negative electrode electrolytic solution 11 were 20 ml and 6 ml, respectively; and the charge/discharge test was carried out at a constant current of 105 mA and with the upper limit voltage being set at 1.3 V and the lower limit voltage being set at 0.7 V. Fig. 11 shows a charge/discharge curve until the fifth cycle of the redox flow battery 10. In the fifth cycle, the coulomb efficiency was 88%; the voltage efficiency, 92%; and the energy density, 0.81 Wh/L.

### [Example 26]

The heterocyclic compound represented by the formula (3-4) was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 12. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 12.

A redox flow battery 12 was fabricated as in Example 22, except for altering the negative electrode electrolytic solution 8 and the positive electrode electrolytic solution 8 in Example 22 to the negative electrode electrolytic solution 12 and the positive electrode electrolytic solution 12, respectively. By using the redox flow battery 12, the charge/discharge test was carried out by the same operation as in Example 22. The solution volumes of the positive electrode electrolytic solution 12 and the negative electrode electrolytic solution 12 were 20 ml and 6 ml, respectively; and the charge/discharge test was carried out at a constant current of 105 mA and with the upper limit voltage being set at 1.6 V and the lower limit voltage being set at 0.5 V. Fig. 12 shows a charge/discharge curve until the fifth cycle of the redox flow battery 11. In the fifth cycle, the coulomb efficiency was 87%; the voltage efficiency, 91%; and the energy density, 0.85 Wh/L.

### [Example 27]

The heterocyclic compound represented by the formula (3-5) was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 13. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 13.

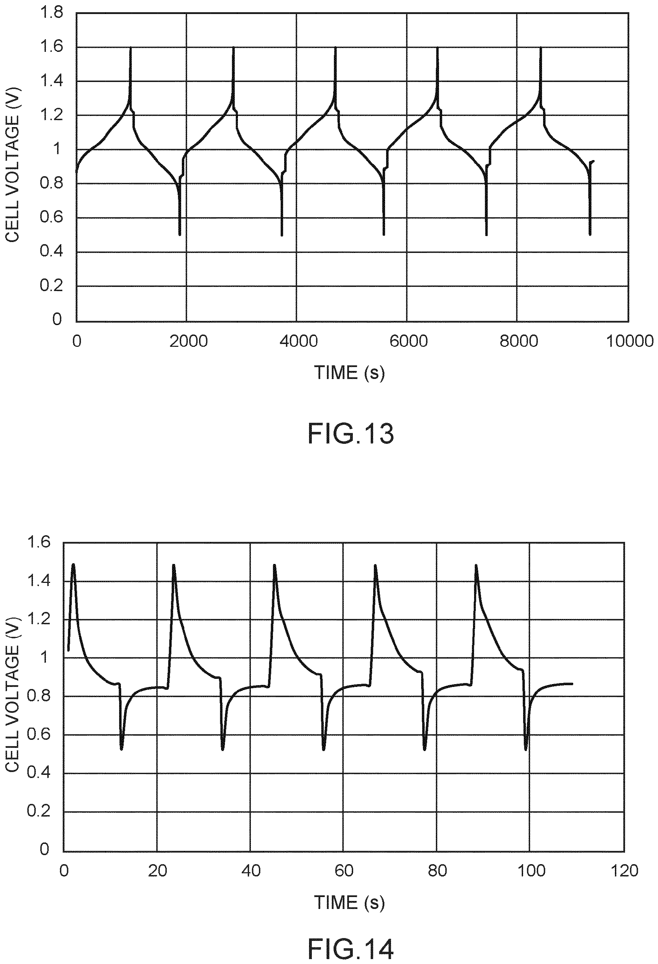

A redox flow battery 13 was fabricated as in Example 22, except for altering the negative electrode electrolytic solution 8 and the positive electrode electrolytic solution 8 in Example 22 to the negative electrode electrolytic solution 13 and the positive electrode electrolytic solution 13, respectively. By suing the redox flow battery 13, the charge/discharge test was carried out by the same operation as in Example 22. The solution volumes of the positive electrode electrolytic solution 13 and the negative electrode electrolytic solution 13 were 20 ml and 6 ml, respectively; and the charge/discharge test was carried out at a constant current of 105 mA and with the upper limit voltage being set at 1.6 V and the lower limit voltage being set at 0.5 V. Fig. 13 shows a charge/discharge curve until the fifth cycle of the redox flow battery 12. In the fifth cycle, the coulomb efficiency was 90%; the voltage efficiency, 86%; and the energy density, 0.94 Wh/L.

### [Comparative Example 1]

Sodium 9,10-anthraquinone-2-sulfonate was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 14. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 14.

A redox flow battery 14 was fabricated as in Example 22, except for altering the negative electrode electrolytic solution 8 and the positive electrode electrolytic solution 8 in Example 22 to the negative electrode electrolytic solution 14 and the positive electrode electrolytic solution 14, respectively. By using the redox flow battery 14, the charge/discharge test was carried out by the same operation as in Example 22. The solution volumes of the positive electrode electrolytic solution 14 and the negative electrode electrolytic solution 14 were 20 ml and 6 ml, respectively; and the charge/discharge test was carried out at a constant current of 105 mA and with the upper limit voltage being set at 1.5 V and the lower limit voltage being set at 0.5 V. Fig. 14 shows a charge/discharge curve until the fifth cycle of the redox flow battery 14. In the redox flow battery 14, the active material concentration was insufficient and good charge/discharge characteristic was not attained.

### [Comparative Example 2]

Sodium 9,10-anthraquinone-1,5-disulfonate was dissolved in a sodium chloride (manufactured by Tokyo Chemical Industry Co., Ltd., purity: >99.5%) aqueous solution (1.0 mol/L) to attain 0.1 mol/L to thereby fabricate a negative electrode electrolytic solution 15. On the other hand, sodium iodide (manufactured by Junsei Chemical Co., Ltd., first grade) was dissolved in a sodium chloride (manufactured by Tokyo Chemical Industry Co., Ltd., purity: >99.5%) aqueous solution (1.0 mol/L) to attain 0.2 mol/L to thereby fabricate a positive electrode electrolytic solution 15.

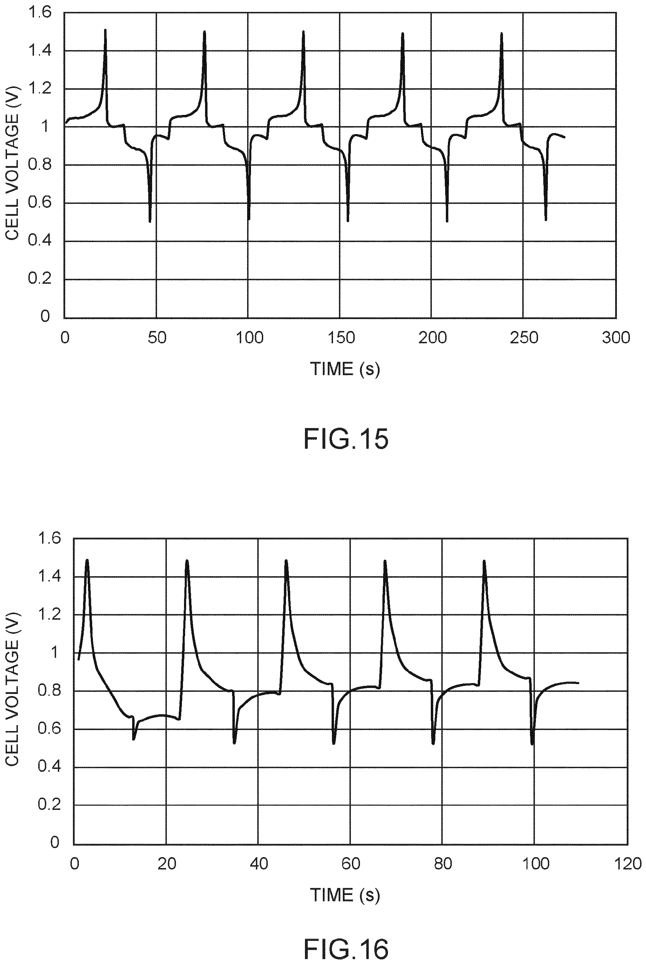

A redox flow battery 15 was fabricated as in Example 22, except for altering the negative electrode electrolytic solution 8 and the positive electrode electrolytic solution 8 in Example 22 to the negative electrode electrolytic solution 15 and the positive electrode electrolytic solution 15, respectively. By using the redox flow battery 15, the charge/discharge test was carried out by the same operation as in Example 22. The solution volumes of the positive electrode electrolytic solution 15 and the negative electrode electrolytic solution 15 were 20 ml and 6 ml, respectively; and the charge/discharge test was carried out at a constant current of 105 mA and with the upper limit voltage being set at 1.5 V and the lower limit voltage being set at 0.5 V. Fig. 15 shows a charge/discharge curve until the fifth cycle of the redox flow battery 15. In the fifth cycle, the coulomb efficiency was 71%; the voltage efficiency, 76%; and the energy density, 0.01 Wh/L. The redox flow battery 15 was inferior in the cycle characteristic and the energy density to the redox flow batteries fabricated in the other Examples.

### [Comparative Example 3]

Potassium 9,10-anthraquinone-1,8-disulfonate was dissolved in a potassium chloride (manufactured by Junsei Chemical Co., Ltd., special grade) aqueous solution (1.0 mol/L) to attain 0.1 mol/L to thereby fabricate a negative electrode electrolytic solution 16. On the other hand, potassium iodide (manufactured by Junsei Chemical Co., Ltd., special grade) was dissolved in a potassium chloride (manufactured by Junsei Chemical Co., Ltd., special grade) aqueous solution (1.0 mol/L) to attain 0.2 mol/L to thereby fabricate a positive electrode electrolytic solution 16

A redox flow battery 16 was fabricated as in Example 22, except for altering the negative electrode electrolytic solution 8 and the positive electrode electrolytic solution 8 in Example 22 to the negative electrode electrolytic solution 16 and the positive electrode electrolytic solution 16, respectively. By using the redox flow battery 16, the charge/discharge test was carried out by the same operation as in Example 22. The solution volumes of the positive electrode electrolytic solution 16 and the negative electrode electrolytic solution 16 were 20 ml and 6 ml, respectively; and the charge/discharge test was carried out at a constant current of 105 mA and with the upper limit voltage being set at 1.5 V and the lower limit voltage being set at 0.7 V. Fig. 16 shows a charge/discharge curve until the fifth cycle of the redox flow battery 16. In the redox flow battery 16, the active material concentration was insufficient and good charge/discharge characteristic was not attained.

**[Table 7]**

| | Battery | Active material | Average discharge voltage (V) | Coulomb efficiency (%) | Voltage efficiency (%) | Energy density (Wh/L) |
|---|---|---|---|---|---|---|
| Example 22 | redox flow battery 8 | formula (3-1) | 1.10 | 84 | 92 | 1.04 |
| Example 23 | redox flow battery 9 | formula (3-2) | 1.06 | 87 | 88 | 1.08 |
| Example 24 | redox flow battery 10 | formula (3-3) | 0.98 | 84 | 82 | 0.46 |
| Example 26 | redox flow battery 11 | formula (2-1) | 0.94 | 88 | 92 | 0.81 |
| Example 26 | redox flow battery 12 | formula (3-4) | 1.09 | 87 | 91 | 0.85 |
| Example 27 | redox flow battery 13 | formula (3-5) | 1 | 90 | 86 | 0.94 |
| Comparative Example 1 | redox flow battery 14 | 9,10-anthraquinone-2-sulfonic acid (Na) | N/A | N/A | N/A | N/A |
| Comparative Example 2 | redox flow battery 15 | 9,10-anthraquinone-1,5-disulfonic acid (Na) | 0.84 | 71 | 76 | 0.01 |
| Comparative Example 3 | redox flow battery 16 | 9,10-anthraquinone-1,8-disulfonic acid (K) | N/A | N/A | N/A | N/A |

As indicated in Table 7 and Figs. 8 to 13, it is clear that the redox flow batteries 8 to 13 fabricated in Examples 22 to 27 had a high average discharge voltage (V) and a high energy density, and had a good cycle characteristic. Here, in the above Table 7, "N/A" represents that a measurement value was non-available.

### Industrial Applicability

An active material containing the heterocyclic compound of the present invention, and an electrolytic solution and a redox flow battery containing the active material can provide the redox flow battery with a high energy density and a good cycle characteristic. Further, the electrolytic solution of the present invention, which is an aqueous electrolytic solution, is safer and easier to handle as compared with organic solvent-based electrolytic solutions, enabling the electrolytic solution to be applied to a wide range of uses.

## Claims

1. A heterocyclic compound represented by the following formula (1), (2) or (3), or a salt thereof: 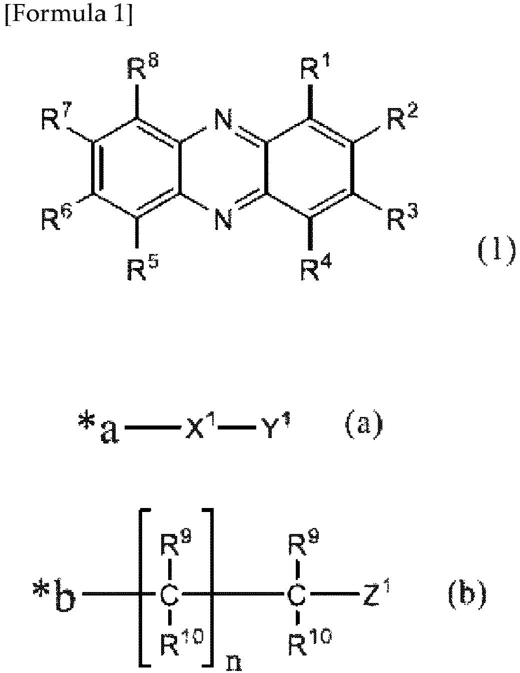 wherein:
R¹ to R⁸ are each independently a hydrogen atom, an acidic group, an alkoxy group, an alkyl group, an amino group, an amide group or a group represented by formula (a), and at least one of R¹ to R⁸ is a group represented by formula (a);
X¹ represents an oxygen atom, a sulfur atom or NY²;
Y¹ is a group represented by formula (b);
Y² is a hydrogen atom, an alkyl group, a carbonyl group, a sulfonyl group or a group represented by formula (b);
R⁹ and R¹⁰ are each independently a hydrogen atom or a substituent; Z¹ is an acidic group;
n is an integer of 1 to 7;
*a represents a bonding site to formula (1); and
*b represents a bonding site to X¹ of formula (a), 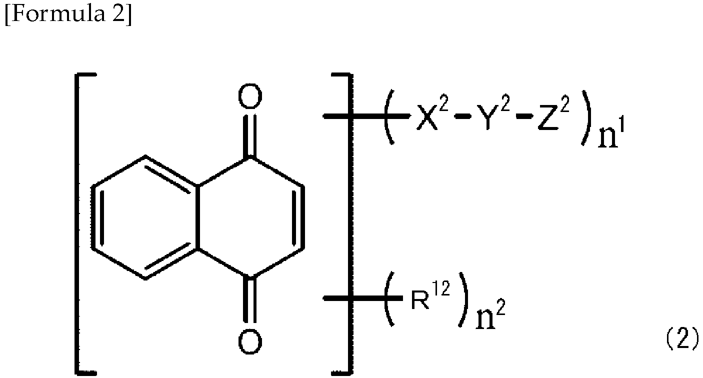 wherein:
X² is an oxygen atom, a sulfur atom or NR¹¹;
Y² is a linker;
Z² is an acidic group;
n¹ is an integer of 1 to 6;
R¹¹ is a hydrogen atom or an alkyl group;
each R¹² is independently an alkyl group or an acidic group; and
n² is an integer of 0 to 5, and 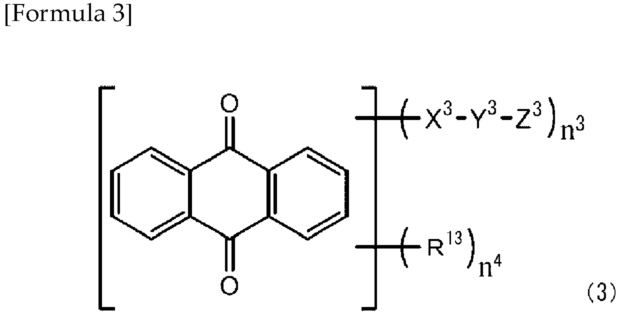 wherein:
X³ is an oxygen atom or a sulfur atom;
Y³ is a linker;
Z³ is an acidic group;
n³ is an integer of 3 to 8;
each R¹³ is independently an alkyl group or an acidic group; and
n⁴ is an integer of 0 to 5.

2. The heterocyclic compound or a salt thereof according to claim 1, wherein Z¹ in the formula (b) is a sulfo group.

3. The heterocyclic compound or a salt thereof according to claim 1 or 2, wherein at least two of R¹ to R⁸ in the formula (1) are each a group represented by the formula (a).

4. The heterocyclic compound or a salt thereof according to any one of claims 1 to 3, wherein R² and R³ in the formula (1) are each a group represented by the formula (a).

5. An active material, comprising at least one heterocyclic compound or a salt thereof according to any one of claims 1 to 4.

6. An electrolytic solution, comprising the active material according to claim 5.

7. The electrolytic solution according to claim 6, wherein the electrolytic solution is an electrolytic solution for a redox flow battery.

8. The electrolytic solution according to claim 6 or 7, wherein a content of water contained in the electrolytic solution is 1% by mass or higher and 99.9% by mass or lower.

9. The electrolytic solution according to claim 8, wherein the content of water contained in the electrolytic solution is 10% by mass or higher and 99% by mass or lower.

10. A redox flow battery, comprising the electrolytic solution according to any one of claims 6 to 9.

11. The redox flow battery according to claim 10, further comprising an electrode and a membrane.
